# EUROPEAN PATENT SPECIFICATION

(11) **EP 0 785 941 B1**
(45) Date of publication and mention of the grant of the patent: **19.04.2000**
(21) Application number: 95938196.3
(22) Date of filing: 13.10.1995
(51) Int. Cl.: C07H 19/04, C07H 21/00, C12Q 1/68, G01N 33/533

(54) **PORPHYRIN LABELING OF POLYNUCLEOTIDES**
PORPHYRIN-MARKIERUNG VON POLYNUKLEOTIDEN
MARQUAGE DE POLYNUCLEOTIDES PAR PORPHYRINES

(30) Priority: 14.10.1994 US 324126
(43) Date of publication of application: 30.07.1997
(73) Proprietor: STRATAGENE, La Jolla, CA 92037 (US)
(72) Inventor: BRAMAN, Jeffrey, C., Carlsbad, CA 92009 (US); MOORES, Jane, C., San Diego, CA 92122 (US); ANDERSON, Jack, D., Oceanside, CA 92054 (US); HUANG, Haoqiang, San Diego, CA 92128 (US)
(74) Representative: Goldin, Douglas Michael
(86) International application number: US9512996
(87) International publication number: WO9611937

(56) References cited:
- EP-A- 0 127 797
- WO-A-90/02747
- US-A- 4 375 972
- CHEMICAL ABSTRACTS, vol. 116, no. 12, 1992 Columbus, Ohio, US; abstract no. 119638t, K.G.SHOIKHET ET AL. 'Synthesis of a Metalloporphyrin Bearing an Isothiocanato Group for Oligonucleotide Labelling.' page 924; column 2; & SIB. KHIM. ZH., vol. 4, 1991 pages 32-35,
- MOLECULAR BIOLOGY, vol. 26, no. 3, 1992 pages 490-496, N.V.LEDOVSKIKH ET AL. 'Oligonucleotide Derivatives Suppress Transcription of Influenza Virus RNA.'
- MOLECULAR BIOLOGY REPORTS, vol. 18, no. 1, 1993 pages 43-47, S.N.KHODYREVA ET AL. 'Comparison of Interactions of 5'-Derivatives of Deoxyoctathymidylate with Human DNA Polymerise alpha and HIV Reverse Transcriptase.'
- NUCLEOSIDES AND NUCLEOTIDES, vol. 10, no. 1-3, 1991 pages 641-643, V.V.VLASSOV ET AL. 'Photoactivatable Porphyrin Oligonucleotide Derivatives for Sequence Specific Chemical Modification and Cleavage of DNA.'
- MOLECULAR BIOLOGY, vol. 27, no. 4, 1993 pages 477-482, I.A.LOKHOVA ET AL. 'Effect of Different Substituents Attached to 5'- and 3'-Termini of the Primer on its Initiating Function in Polymerization Catalyzed by AMV Reverse Transcriptase.'

## Description

This invention pertains to the field of porphyrin labeled nucleosides, porphyrin labeled nucleotides, porphyrin labeled oligonucleotides, porphyrin labeled polynucleotides, and methods for the synthesis and use of these compounds.

### BACKGROUND OF THE INVENTION

Nucleic acid hybridization with a labeled probe is the predominant method of detecting the presence of a complementary target sequence in a complex nucleic acid mixture. Several labels useful for incorporation into polynucleotide probes have been described in the prior art.

To date, the most commonly used labels have been radioisotopes such as ³²P, ³⁵S, ³H, ¹⁴C, and ¹²⁵I. Details on the preparation and use of isotopically labeled probes are available in Sambrook *et al.*, Molecular Cloning Manual, 2nd ed., Cold Spring Harbor Laboratory, Cold Spring Harbor, New York (1989) and Ausubel *et al.,* Current Protocols in Molecular Biology, John Wiley & Sons, New York (1987) (continually updated). Isotopic labels' primary advantage is sensitivity. For example, probes labeled with ¹²⁵I have been detected at levels as low as 1.6 attomole (1.6 x 10⁻¹⁸ mol), Langdale and Malcolm, Gene 36:201 (1985).

However, despite this sensitivity advantage, isotopic labels suffer from numerous disadvantages. For example, the sensitive detection of a signal typically requires very long autoradiograph exposure times. Methodology employing radiolabels is time consuming, labor intensive, expensive, and hazardous. Additional disadvantages of isotopic labels include the short half-life of the most commonly used isotope, ³²P (t_{½} ≈ 14.3 days), necessitating frequent preparations of probes. The shelf-life of radiolabeled probes is also reduced by self-destruction induced by the radiolabel.

To overcome the shortcomings of isotopic labels, non-isotopic labels have been developed and they have been described in the literature. Non-isotopic labels are characterized as being either direct or indirect. Direct labels act as reporter groups by generating a detectable signal. Indirect labels do not generate signals, and must be coupled with a second moiety that bears a signal-generating reporter group.

The most commonly used direct non-isotopic labels are fluorophores such as fluorescein and rhodamine. The ability to attach fluorescent dyes to different probe molecules allows the simultaneous detection of hybridization or biosynthetic products involving more than one probe. Both automated fluorescent DNA sequencing and restriction endonuclease fingerprinting have been carried out using fluorophores. See, *e.g.* Prober *et al*., Science 233:336 (1987); Smith *et al*., Nature 321:674 (1986).

While safe and convenient to use, direct labels such as fluorophores suffer from sensitivity problems due in part to high background from intrinsic fluorescence in the sample. Additionally, fluorescence energy transfer (quenching) can reduce signal intensity. Although schemes have been developed to increase the sensitivity of fluorophores, *e.g.* delaying fluorescence with europium or terbium attached to proteins through chelators, Dahlen, Anal. Biochem. 164:78 (1987), these methods have not attained widespread use and require difficult manipulations.

Indirect labels incorporated into polynucleotide probes must be detected following binding of the label to a signal generating system. Biotin labels are the most widely used indirect labels. Following incorporation of biotin into a polynucleotide probe, biotin is usually detected by bringing the biotin into contact with avidin or streptavidin that is covalently linked to a reporter group such as an enzyme, fluorescent moiety, or luminescent moiety. Enzyme labels provide the most sensitive non-isotopic signals due to the amplification provided by the enzyme.

The most commonly used avidin or streptavidin linked reporter groups are the enzymes alkaline phosphatase and horseradish peroxidase. Renz and Kurz, Nucl. Acids Res.12:3435 (1984) and Jablonski *et al.*, Nucl. Acids Res. 14:6115 (1986). Jablonski *et al.,* Nucl. Acids Res. 14:6115 (1986). Horseradish peroxidase and alkaline phosphatase have also been directly attached to probes for an assay using luminol, H₂O₂, and either para-iodophenol or hydroxycinnamic acid, Pollard-Knight *et al.,* Anal. Biochem. 185:84 (1990), to produce a chemiluminescent signal.

The chemiluminescent labels isoluminol and acridinium have been directly attached to probes, Urdea *et al.*, Nucl. Acids Res. 16:4937 (1988) and Septak, J. Biolumin. Chemilumin. 4:357 (1989). However, the sensitivity of these labels was lower than that achieved by enzyme reporters. The directly attached isoluminol and acridinium produced a chemiluminescent signal limited to one photon per reporter group, *see* Urdea *et al*., Nucl. Acids Res. 16:4937 (1988). Indirect detection of the directly attached chemiluminescent substrates with alkaline phosphatase following blotting produced a signal sufficiently sensitive to substitute for radiolabels in DNA sequencing, *see* Tizard *et al.*, Proc. Natl. Acad. Sci. USA 87:4514 (1990).

Although indirect detection schemes involving enzymes can be as sensitive as direct isotopic labels, the methods are time consuming and labor intensive. These methods typically involve blocking, reaction with a reporter conjugate and washing steps that require labor intensive manipulations. Additionally, enzymes may not be stable to the high temperatures often used in hybridization assays.

Accordingly, there continues to exist a need for safe, simple, non-isotopic labeling methods and reagents to directly label polynucleotide probes that have sensitivity levels comparable to those achieved with direct radiolabels or indirect enzyme-based labels.

The present invention overcomes limitations inherent in previously used labeling methods and reagents. The present invention involves porphyrin labeled nucleotides (and structurally related molecules) useful to directly label polynucleotides with a porphyrin reporter moiety.

Porphyrins, a class of small molecules that have the capability to act as catalytic centers, have long been used as catalysts for oxidation reactions in organic synthesis. Porphyrins are analogs of the prosthetic group of heme-containing enzymes that selectively catalyze various biological oxidation reactions.

Examples can be found in the literature where porphyrin moieties have been associated with DNA. *See, e.g.* Meunier, Chem. Rev. 92:1411 (1992) and references cited therein; Le Doan *et al.,* Nucl. Acids Res. 15:8643 (1987); and Le Doan *et al.*, Bioconjugate Chem. 1, 108 (1990). In these procedures, porphyrin-modified polynucleotides were prepared for the purpose of modifying (*e.g.* cleaving) adjacent DNA strands after hybridizing the labeled polynucleotides to complementary DNA. Ci *et al.*, Anal. Chem. Acta 282:695 (1993) studied the interactions of porphyrin with nucleic acids. However, this study involved non-covalent interactions between the porphyrins and the DNA, and did not involve polynucleotide probes labeled with porphyrin as a reporter group. In these cases the porphyrin moiety was not used for detection, and no report was made of a porphyrin-labeled mononucleotide. Several publications describe cationic porphyrins covalently linked to the termini of polynucleotides in order to induce cleavage of a polynucleotide, *e.g.* Le Doan, *et al.,* Biochemistry, 25:6736-6739 (1986), Le Doan, *et al.,* Nucl. Ac. Res. 15:8643-8659 (1987), Casas, *et al.*, Biconjugate Chem. 4:366-371 (1993).

Porphyrins have been used as a signal source in antibody immunoassays. For example, U.S. Patent No. 4,375,972 to Forgioue *et al.* describes a method whereby antibodies and antigens present in small amounts in body fluids are detected and quantified with an antibody-porphyrin conjugate. U.S. Patent 4,614,723 describes the direct detection of porphyrin-labeled antibodies. Motsenbocker *et al.*, Anal. Chem 65:397 (1993) describes a porphyrin chemiluminescence system that is said to be applicable in immunoassays. However, none of these publications discloses the use of porphyrin labeled nucleotides to label polynucleotide probes.

Porphyrins make ideal non-isotopic polynucleotide labels for several reasons. These reasons include (1) the porphyrin moiety can catalyze certain chemical reactions (*e.g.* the oxidation of chemiluminescent substrates such as luminol), making it functionally similar to a reporter enzyme such as horseradish peroxidase; (2) the porphyrin moiety is relatively small in size (mol wt. ≈ 1Kd), and when attached to a nucleotide does not unduly perturb the nucleotide structure, allowing it to be incorporated into nascent polynucleotides by polymerases, and to act as a substrate for other nucleotide-dependent enzymes; (3) porphyrin labels are more stable than commonly used isotopic labels such as ³²P, obviating the need for frequent probe preparation; (4) porphyrin labels are stable to the high temperatures often used in hybridizations; and (5) porphyrin labels do not induce probe degradation to the same extent that isotopic labels induce degradation.

The present invention provides, for the first time, porphyrin labeled nucleosides, nucleotides, oligonucleotides, polynucleotides and various derivatives thereof. The compositions and methods of the present invention provide numerous benefits over previously used techniques for labeling polynucleotides and can be advantageously substituted for previously used labels and labeling methods.

### SUMMARY OF THE INVENTION

The present invention provides various porphyrin labeled compounds and reagents for labeling molecules with porphyrins. The porphyrin labeled compounds include porphyrin labeled nucleosides, nucleotides, polynucleotides and the like. The porphyrin labeled compounds of the invention are designed so that the porphyrin moiety on the labeled compound may be detected on the basis of a reaction product produced by a porphyrin catalyzed oxidation of a porphyrin detection reagent. The oxidation of the porphyrin detection reagent may result in the formation of light, *i.e.*, a chemiluminescent reaction, or a colored compound, *i.e.,* a colorimetric reaction.

One aspect of the invention is to provide compounds having the general structure of:

porphyrin-linker-base-sugar-(phos)ₙ-OH (I)

The terms phos and (phos)ₙ, wherein n is 0-5, and phos may be any of a variety of moieties selected from the group consisting of phosphate, phosphorothioates, phosphonates, or structures represented by the formula 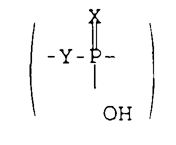 wherein X is O or S,
and Y is selected from the group consisting of O, S, CH₂-, CF₂- and NH-.

Preferred porphyrin labeled compounds provided by the invention include porphyrin labeled mononucleotides (and the corresponding ribonucleotides) such as Mn *tetrakis*(carboxyphenyl) porphyrin-12-dUTP, Mn-*tris*(sulfonatophenyl)-(carboxyphenyl) porphyrin-12-dUTP, Mn-*tris*(sulfonatophenyl)-carboxyphenyl porphyrin-24-dUTP, Mn-*tris*(sulfonatophenyl)-carboxphenyl porphyrin-12-ddUTP, Mn-*tris*(sulfonatophenyl)-carboxyphenyl porphyrin-24-ddUTP and Mn-*tris*(sulfonatophenyl)-carboxyphenyl porphyrin-12-dCTP. However, those skilled in the art will appreciate that numerous variants of the preferred compounds may be synthesized including, for example, changes in the metal ion, the linker, the base, the porphyrin and substituents thereof.

Another aspect of the invention is to provide methods of labeling polynucleotides by covalently attaching a porphyrin to a polynucleotide. The labeling methods described herein include labeling through enzymatic synthesis of polynucleotides with porphyrin labeled polynucleotide precursors, *e.g.* porphyrin labeled nucleoside triphosphates, the direct chemical synthesis of porphyrin labeled polynucleotides, and the mixing of polynucleotides with porphyrin labeling reagents.

Another aspect of the invention is to provide methods of detecting a polynucleotide of interest. Certain polynucleotide detection methods of the invention comprise the step of adding a porphyrin detection reagent so as to detect porphyrin labeled polynucleotides, preferably by a chemical reaction catalyzed by porphyrin moieties. The detection methods of the invention may further comprise the step of performing a nucleic acid hybridization with a porphyrin labeled polynucleotide capable of hybridizing to a polynucleotide target of interest. The hybridization step of the polymerase chain reaction (PCR), *i.e.*, the annealing step, may be adapted so as to employ porphyrin labeled primers for incorporation into the PCR amplification product.

Another aspect of the invention is to provide kits for producing porphyrin labeled polynucleotides. Such kits may include one or more of the following items: porphyrin labeled nucleotides of the invention, nucleoside triphosphates, enzymes having nucleotide polymerase activity, buffers, porphyrin detection reagents, and porphyrin labeled polynucleotide molecular weight standards. Similarly, another aspect of the invention is to provide kits for detecting the presence of polynucleotides of interest by using a porphyrin labeled polynucleotide.

### DESCRIPTION OF THE SPECIFIC EMBODIMENTS

### Definitions

The term "nucleoside," as used herein, refers to a compound comprising a purine or pyrimidine base (or analog thereof) covalently joined to a 5 carbon cyclic sugar (furanose), *e.g.* ribose, 2'-deoxyribose, and 2',3'-dideoxyribose.

The term "nucleotide," as used herein, refers to compounds comprising a nucleoside covalently joined to a phosphate moiety or functional equivalent thereof.

The term "polynucleotide," as used herein, refers to polymers comprising two or more nucleoside moieties, wherein each nucleoside moiety is joined to one (terminal) or two (internal) other nucleoside moieties through phosphate linkages, peptide linkages, phosphonate linkages, phosphorothioate linkages, and the like. RNA and DNA are examples of polynucleotides. The term "polynucleotide", as used herein, unless explicitly noted otherwise, refers to molecules comprising a plurality of nucleosides joined by one of a variety of linkages, phosphate, or otherwise so as to maintain the spatial relationship between bases present in a naturally occurring polynucleotide.

The term "oligonucleotide", as used herein, refers to relatively small polynucleotides, *e.g.*, polynucleotides of between 2 and about 35 base pairs in length.

### THE INVENTION

The invention described herein provides numerous different porphyrin labeled nucleosides, porphyrin labeled nucleotides or porphyrin labeled polynucleotides and various structurally related molecules. The porphyrin label may be detected by chemiluminescent or colorimetric reactions catalyzed by the porphyrin label. The novel porphyrin labeled compounds provided herein may have a variety of structures such as DNA, RNA, and non-naturally occurring polynucleotide derivatives such as PNAs (peptide nucleic acids), phosphonates, phosphorothioates, and the like.

The invention provides numerous embodiments of porphyrin labeled nucleosides, porphyrin labeled nucleotides, and porphyrin labeled polynucleotides, comprising a porphyrin labeled nucleoside moiety having the structure represented by the following formula:

porphyrin-linker-base-sugar-(phos)ₙ-OH (I)

Thus compounds of formula I comprise five moieties, (1) porphyrin, (2) a linker, (3) a base, (4) a sugar and (5) (phos)ₙ, wherein n is 0-5. The hyphens in formula (I) represent covalent linkages between the indicated moieties. The exact site of the linkage may vary with the identity of the structure of the particular moiety.

The "sugar" moiety of the compound of formula (I) corresponds to a carbohydrate that may serve the structural and chemical functions of a nucleoside sugar such as ribose, deoxyribose, acyclic analogs of ribose and deoxyribose, carbocyclic analogs of ribose and deoxyribose, cyclic analogs of ribose and deoxyribose having extra heteroatoms, *e.g.*, dioxolane, dithiolane, and oxathiolane derivatives, and the like.

The "(phos)ₙ" moiety may be any of a variety of moieties selected from the group consisting of phosphate, phosphorothioates, phosphonates, or structures represented by the formula 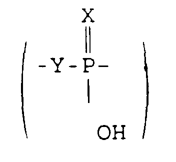 wherein X is O or S,
and Y is selected from the group consisting of O, S, CH₂-, CF₂- and NH-. The "(phos)ₙ" moiety is covalently linked to the sugar moiety in such a way as to be structurally equivalent to the native structure of nucleic acids, i.e., structurally equivalent to attachment of the phosphate group to the 5' position of the furanose ring. The term "n" indicates that 0 to 5 (phos) moiety subunits may be present. When "n" is 0, the (phos) moiety is absent.

The "base" moiety of the compound of formula (I) corresponds to a heterocyclic nucleotide base found in naturally occurring nucleic acids such as adenine, cytosine, hypoxanthine, uracil, thymine, guanine and analogs thereof, including non-naturally occurring bases that are capable of forming base-pairing relationships with naturally occurring nucleotide bases. Such non-naturally occurring heterocyclic bases include, but are not limited to, aza and deaza pyrimidine analogs, aza and deaza purine analogs as well as other heterocyclic base analogs, wherein one or more of the carbon and nitrogen atoms of the purine and pyrimidine rings have been substituted by heteroatoms, *e.g.* oxygen, sulfur, selenium, phosphorus, and the like. Additionally, other heterocyclic ring systems include 7-deazapurine analogs, *e.g.* 7-deazaadenine, 7-deazaguanine, and 7-deazahypoxanthine; 9-deazapurine analogs, *e.g.* 9-deazaadenine, 9-deazaguanine, and 9-deazahypoxanthine; 8-azapurine analogs, *e.g.* 8-azaadenine, 8-azaguanine, and 8-azahypoxanthine, 5-azapyrimidines (1, 3, 5-triazines), *e.g.* 5-azacytosine, 5-azathymine, and 5-azauracil, 6-azapyrimidines (1, 2, 4 triazines), *e.g.* 6-azacytosine, 6-azathymine, and 6-azauracil; dideaza-heterocyclic base analogs, *e.g.* 8 aza-7-deazapurines (pyrazolo-[3, 4-d] pyrimidines), amino-imidazole carboxamide, appropriately derivatized 1, 2, 4-triazoles, thiazoles; selenazoles; methylated base analogs, *e.g.* 5 methylcytosine, and the like. Other examples of non-naturally occurring bases that may serve as the base in the compound of formula I include: 5-fluorouracil, 5-bromouracil, 5-chlorouracil, 5-iodouracil, 5-(carboxyhydroxylmethyl) uracil, 5-carboxymethylaminomethyl-2-thiouridine, 5-carboxymethylaminomethyluracil, methylpseudouracil, N2, N2-dimethylguanine, 2-methyladenine, 2-methylguanine, 4-N-methylcytosine, 5-methylcytosine, N6-methyladenine, 5-methylaminomethyluracil, 5-methoxyaminomethyl-2-thiouracil, 5'-methoxycarbonylmethyluracil, uracil-5-oxyacetic acid (v), pseudouracil, 2-thiocytosine, 2-thiouracil, 4-thiouracil, 5-methyluracil, and 2, 6-diaminopurine. Preferred base moieties are those bases that may be incorporated into one strand of double-stranded polynucleotides so as to maintain a base pairing structural relationship with a naturally occurring base on the complementary strand at the double-stranded polynucleotide. Suitable bases contain a site for attachment to the linker, such that the linker does not substantially interfere with base pairing relationships or function of the enzyme selected for use in the given method. Preferred embodiments of the invention include compounds where the linker moiety is attached at position C-5, C-6, or N-4 of pyrimidine bases, at position C-8, N-6, or N-2 of purine bases, and at positions C-8, C-7, N-6, or N-2 of deazapurines.

The three dimensional structure of double-stranded polynucleotides is very well understood. A person skilled in the art could readily select suitable base moieties for the compound of formula I. Accordingly, the person skilled in the art may readily select base moieties that are functionally equivalent to naturally occurring polynucleotide bases and at the same time a suitable site for attachment of the linker. The heterocyclic base is joined to the sugar through a covalent glycosidic linkage.

The "linker" moiety of the compound of formula (I) corresponds to an essentially linear molecule of variable length that serves to join the heterocyclic base moiety to a porphyrin moiety. Additionally,the linker functions as a spacer so as to position the porphyrin moiety a sufficient distance from the other portions of the molecule in order to avoid steric hinderance problems. The linker may consist of any of a variety of structures. The linker may vary considerably in length. Preferably, the backbone of the linker (the straight chain portion) contains 1 to 50 atoms. Suitable linkers for use in the invention include linkers as described in U.S. Patents 5,047,519 and 5,151,507. Other suitable linkers include moieties having the structure: -CH₂-(CH₂-CH₂)ᵥ-CH₂-NHC(O)-Q-, -CH₂-(CH₂-CH₂)ᵥ-CH₂-C(O)-NH-C(O)-Q-, -S-CH₂C(O)-Q-, -S-CH₂CH₂NH-C(O)-Q-, -O-CH₂C(O)-Q-, -O-CH₂CH₂NH-C(O)-Q-,

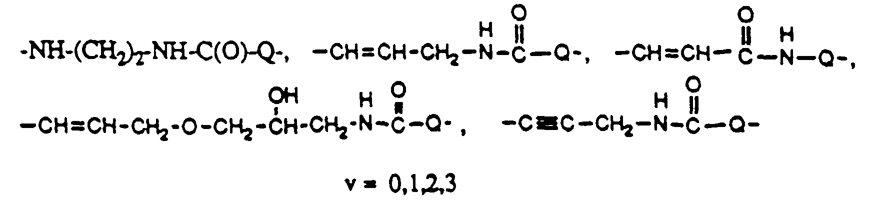

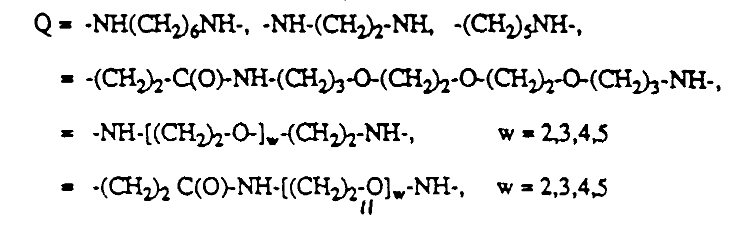

However, one skilled in the art having the benefit of this disclosure will appreciate that many alternative linker structures may be utilized in the invention, provided they are able to join a given porphyrin to the other portions of the molecule according to formula I without substantially interfering with base pairing relationships or function of the enzyme selected for use in the given method.

The "porphyrin" moiety of the compound of formula (I) may be any of a number of molecules having a porphyrin structure (e.g. substituted porphines or phthalocyanines) that are capable of complexation of with metal ions. Unless indicated otherwise, the term "porphyrin" as used herein refers to both porphyrins and the corresponding metal porphyrins. Details on the synthesis of individual porphyrin molecules can be found by consulting the chemical literature, *e.g.* chemical abstracts and comprehensive treatises such as The Porphyrins (Volumes 1-7), D. Dolphin Ed. (Academic Press, New York, 1978). The porphyrin moiety on the compound of formula I preferably contains phenyl groups attached to each of the four *meso* positions, more preferably, each phenyl ring is substituted with electron-withdrawing groups, such as sulfate groups, carboxylate groups, and the like. The porphyrin moiety has the general formula: 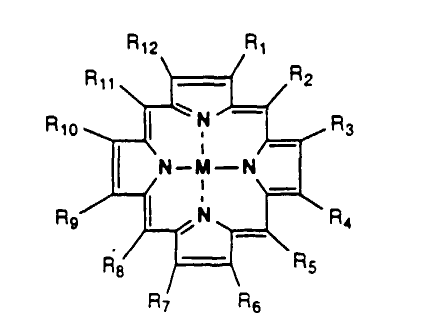
- M: = Mn, Fe, Zn, Co, Ni, Cu, Cr, (as coordinated metal ions). 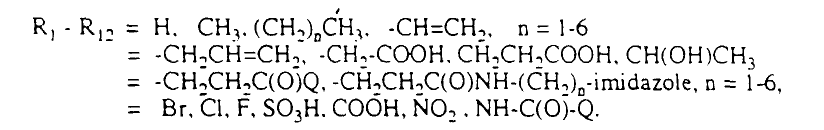 R₂ = R₅ = R₈ = R₁₁ = aryl, substituted aryl
= structures **7, 8** 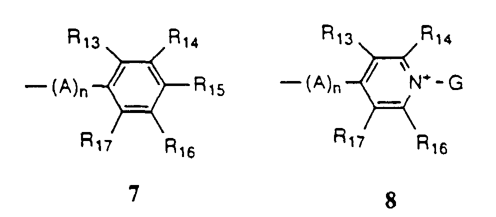
- R₁₃ - R₁₇: = H, Br, Cl, F, SO₃H, CO₂H, NO₂, C(O)-Q-,
= NH-C(O)-Q-, -C(O)-(CH₂)ₙ-imidazole, NH-C(O)-(CH₂)ₙ-imidazole
= NH-T, T = -(CH₂)ₙCH₃, -(CH₂)ₙ-imidazole, n = 1-6
A = -(CH₂₋)ₘ, -(CH=CH-)ₘ, -(C≡C-)ₘ, m = 0, 1, 2, 3
G = -(CH2)ₘ-CH₃, -(CH₂)ₘ-Q- m = 0, 1, 2, 3

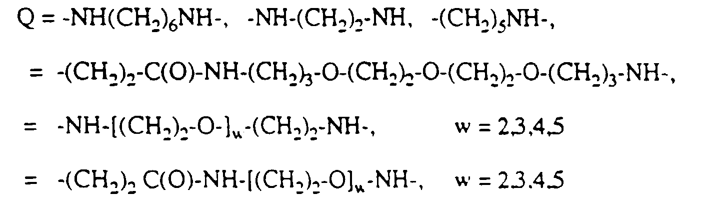

When the (phos)ₙ moiety of the compound of formula (I) is a triphosphate, preferred embodiments of the compound of formula (I) have the structure: 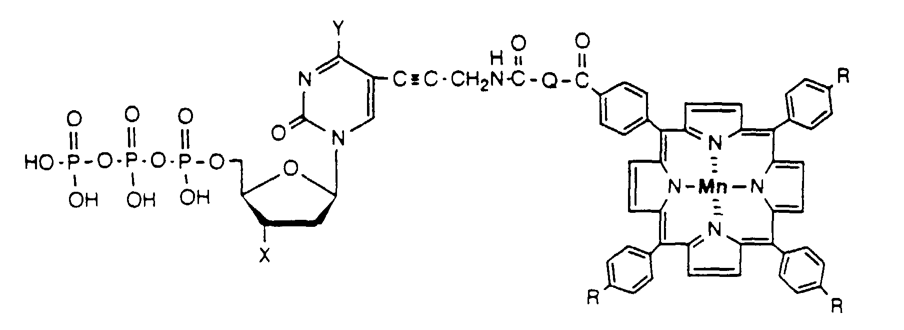
1 Mn-*tetrakis*(carboxyphenyl)porphyrin-12-dUTP (R - COOH, X,Y = OH, Q = -(CH₂)₅ -NH-).
2 Mn-*tris*(sulfonatophenyl)-carboxyphenyl porphyrin-12-dUTP (R = SO₃H, X,Y = OH, Q = -(CH₂)₅-NH-).
3 Mn-*tris*(sulfonatophenyl)-carboxyphenyl porphyrin-24-dUTP (R = SO₃H, X,Y = OH, O = -NHC(O)(CH₂)₂C(O)NH(CH₂)₃O(CH₂)₂O(CH₂)₂O(CH₂)₃)NH-).
4 Mn-*tris*(sulfonatophenyl)-carboxyphenyl porphyrin-12-ddUTP (R = SO₃H, X = H, Y = OH, Q = -(CH₂)₅-NH-).
5 Mn-*tris*(sulfonatophenyl)-carboxyphenyl porphyrin-24-dUTP (R = SO₃H, X = H, Y = OH, Q = -NHC(O)(CH₂)₂C(O)NH(CH₂)₃O(CH₂)₂O(CH₂)₂O(CH₂)₃)NH-).
6 Mn-*tris*(sulfonatophenyl)-carboxyphenyl porphyrin-12-dCTP (R = SO₃H, X = OH, Y = NH₂, Q = -(CH₂)₅-NH-).

Additional preferred embodiments include the corresponding adenosine and guanosine analogs. The compounds of formula I may be made using organic synthesis techniques well known to the person of ordinary skill in the art. By referring to the working examples provided herein, and also by referring to well known techniques in organic chemistry, the person of ordinary skill in the art may produce numerous variants of the exemplified compounds.

The invention also provides for various compounds that comprise a porphyrin labeled nucleoside moiety of formula (I) such as nucleoside phosphoramidites, nucleoside triphosphates, polynucleotides, and the like. The compounds of the invention include porphyrin labeled nucleoside derivatives adapted for use in solid phase polynucleotide synthesis, *e.g.* phosphoramidite derivatives. A description of phosphoramidites, their synthesis, and use in polynucleotide synthesis can be found, among other places, in Oligonucleotides and Analogues: A Practical Approach, ed. Eckstein, IRL Press, Oxford (1992).

The invention also provides various porphyrin labeled polynucleotides that comprise as part of their structure at least one of the porphyrin labeled compounds of formula (I) and a second nucleoside moiety (either unlabeled or labeled with porphyrins) joined by linkages such as a phosphate linkage (as in naturally occurring polynucleotides), phosphorothioate linkages, peptide linkages (as in PNAs), phosphonate linkages, and the like. These various linkages are represented, in part, by the "phos" moiety in the compounds of formula I. The linkages between individual nucleoside moieties in the porphyrin labeled polynucleotides of the invention are selected so as to maintain a spatial relationship between the bases suitable to allow base pair formation in a double-stranded polynucleotide. The porphyrin labeled polynucleotides of the invention may be of virtually any length.

The porphyrin labeled polynucleotides of the invention are useful in a variety of applications, in particular, as hybridization probes for the detection of polynucleotides of interest. Polynucleotides of interest for detection by porphyrin labeled hybridization probes include virtually any polynucleotide that is suitable for detection with conventional polynucleotides, *i.e.*, isotopically labeled polynucleotides. Among the many other uses of porphyrin labeled polynucleotides are as signal sources in DNA footprinting studies, DNA gel shift assays, detection of DNA sequencing ladders, and for detecting PCR amplification products.

The porphyrin labeled polynucleotides of the invention may be conveniently produced by incorporating one or more porphyrin labeled nucleotides, *e.g.* compounds of formula I wherein (phos)ₙ represents a triphosphate, into polynucleotides produced by a polymerase mediated synthesis reaction. As with conventional nucleoside triphosphates, the porphyrin labeled nucleoside triphosphates of the invention may be readily incorporated into polynucleotides of varying lengths by enzymatic reactions that are well known to the person of ordinary skill in the art.

For example, polynucleotide probes can be labeled by a porphyrin at many bases within a polynucleotide by incorporating the porphyrin labeled nucleosides of the present invention into a polynucleotide during the process of synthesis of the polynucleotide. The porphyrin labeled polynucleotide may be synthesized by a variety of techniques that are also useful for incorporating nucleotides, via nucleoside triphosphate intermediates, into polynucleotides. These techniques are well known to the person of ordinary skill in the art of molecular biology. These techniques include nick translation, as described in Rigby *et al.*, J. Mol. Biol. 113:237 (1977), incorporated herein by reference; random priming as described in Feinberg and Vogelstein, Anal. Biochem. 132:6 (1983) and Feinberg and Vogelstein, Anal. Biochem. 137:266 (1984), incorporated herein by reference; and PCR (polymerase chain reaction) as described in Saiki *et al.* Science 239:487 (1988); Langer *et al.,* Proc. Natl. Acad. Sci. USA 78:6633 (1981); and Day *et al.*, Biochem. J. 267:119 (1990), incorporated herein by reference.

Porphyrin labeled polynucleotide probes may also be prepared by primer extension from a single-stranded DNA template, *e.g.* M13, by run-off polymerization or asymmetric PCR using Taq DNA Polymerase as described in Stürzl and Roth, Anal. Biochem. 185:164 (1990), incorporated herein by reference.

Similarly, porphyrin labeled RNA probes may be produced that are labeled at many base positions by incorporating the porphyrin labeled nucleotides of the present invention by in *vitro* transcription as described in Green *et al.,* Cell 32:681 (1983); Tabor and Richardson, Proc. Natl. Acad. Sci. USA 82:1074 (1985); and Langer *et al.,* Proc. Natl Acad. Sci. USA 78:6633 (1981), incorporated herein by reference.

The porphyrin labeled nucleotides of the invention may also be used for labeling in the reactions in which the 3'-end(s) of double stranded DNA may be labeled by filling in with *E. coli* DNA polymerase I (Klenow fragment), as described in Langer *et al.,* Proc. Natl. Acad. Sci. USA 78:6633 (1981) and Delius *et al.,* Nucl. Acids Res. 13:5457 (1985), incorporated herein by reference, or by replacement synthesis using T4 DNA polymerase as described in Langer *et al., supra.*

The porphyrin labeled nucleotides of the invention may be used for labeling in the reactions in which the 3'-end of a polynucleotide may be labeled with terminal transferase, as described in Kumar *et al.*, Anal. Biochem. 169:376 (1988), incorporated herein by reference. The porphyrin labeled nucleotides of the invention may also be used to terminate chain elongation when a 3' hydroxyl group is missing from the sugar on the nucleotides as described in Prober *et al.,* Science 238:336 (1987), incorporated herein by reference.

In addition to providing for the enzymatically mediated synthesis of porphyrin labeled polynucleotides, the porphyrin labeled polynucleotides of the invention may also be produced by chemical labelling methods. For example, porphyrin labeled polynucleotides may be produced by (1) reacting a porphyrin analog, e.g., an activated ester, with a polynucleotide that has been modified to contain a reactive amine or thiol group, or (2) by use of a porphyrin labeled phosphoramidite reagent in solid phase synthesis of polynucleotides (such solid phase synthesis may be automated).

While these examples illustrate the many uses of the porphyrin labeled nucleosides and polynucleotides of the present invention, the person of ordinary skill in the art will appreciate that virtually any method that involves chemical or enzymatic incorporation of nucleosides, deoxynucleoside triphosphates, and analogs thereof, can be used to incorporate the porphyrin labeled nucleosides of the present invention into polynucleotides.

The amount of porphyrin label incorporated into a porphyrin labeled polynucleotide of the invention synthesized enzymatically may be conveniently controlled by varying the number of polynucleotide precursors that are labeled with porphyrins. For example, a porphyrin labeled polynucleotide synthesized from a template by a polymerase catalyzed polymerization reaction may contain more porphyrins when two of the nucleotide precursors, *e.g.* dCTP and the TTP, are porphyrin labeled compounds of the invention, than when only one of the polynucleotide precursors is a porphyrin labeled compound of the invention. Additionally, the desired number of porphyrins on a given polynucleotide may be attained by performing a polynucleotide synthesis reaction using a mixture of porphyrin labeled polynucleotide precursors, *e.g.* porphyrin labeled dCTP, and the corresponding unlabeled polynucleotide precursor, *e.g.* dCTP.

The porphyrin moieties on a porphyrin labeled polynucleotide of the invention and, therefore, the attached polynucleotide, may be detected by chemiluminescent or colorimetric chemical reactions catalyzed by the porphyrin moiety, wherein the metal porphyrin acts to catalyze an oxidation reaction. The substrates for such reactions are referred to herein as "porphyrin detection reagents." The porphyrin catalyzed reactions in which the porphyrin detection reagent is oxidized are referred to herein as "porphyrin detection reactions." However, the porphyrin moiety can also be detected by porphyrin-specific immunoreagents, such as porphyrin-specific antibodies, or spectroscopically.

The porphyrin moiety in the compounds of the invention may be any of a variety of different porphyrin molecules capable of acting as a catalyst to oxidize reactants so as to produce a detectable signal, such as in a chemiluminescent or colorimetric reaction. Chemiluminescent reactions catalyzed by metal porphyrins are described in Motsenbocker, *et al.*, Anal. Chem. 65:397-402 (1993). The metal porphyrins described in Motsenbocker *et al.* are particularly preferred as porphyrin moieties in the compounds of formula (I) because, among other reasons, they can catalyze a porphyrin detection reaction in the absence of hydrogen peroxide. Other suitable porphyrin moieties and porphyrin detection reactions are described, among other places, in Saito *et al.* Chem. Pharm. Bulletin 7:2885 (1986). In order to determine whether a given porphyrin is suitable for use as a porphyrin moiety of the compounds of the invention, the porphyrin, corresponding porphyrin labeled nucleoside or corresponding porphyrin labeled polynucleotide, may be readily tested to determine if the particular porphyrin can catalyze the oxidation of reactants (porphyrin detection reagents) producing an easily detectable product such as light or a colored reaction product. Examples of such reactions and porphyrin detection reagents for these reactions are well known in the art. Such reactions include, for example, the chemiluminescent reactions of the type described in Motsenbocker, *et al. supra, e.g.* using luminol, isoluminol, and other cyclic hydrazides. Suitable compounds for use as porphyrin detection reagents include compounds having the formula: 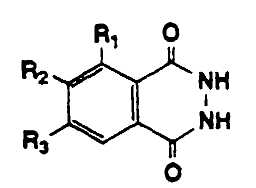 R₁, R₂, R₃ = H, NH₂, NZ₂ Z = H, alkyl 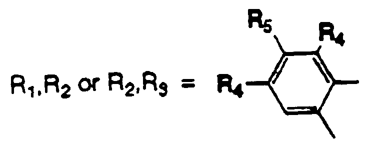 R₄, R₅ = H, NH₂, NZ₂ Z = H, alkyl
Other types of porphyrin detection reagents are compounds that produce a detectable colored reaction product when oxidized by a metal porphyrin and include well known colorimetric substrates for peroxidase enzymes. These colorimetric substrates include, 3,3'-diaminobenzidine, 4-chloro-1-naphthol, and 3-amino-9-ethylcarbazole, and the like. Manganese is particularly preferred for use as the metal in the metal porphyrin when the detection reaction is a chemiluminescent reaction of the type described in Motsenbocker, *et al., supra.* The methods of chemical synthesis of many porphyrins are known to the person of ordinary skill in the art of organic chemistry.

When the porphyrin detection reagent is a substrate in a porphyrin catalyzed chemiluminescent reaction, the reaction preferably takes place in a high pH solution. Preferably the pH of the solution in which the chemiluminescent reaction takes place is about 13. The high pH of the solution causes the majority of luminol (or structurally related porphyrin detection reagents) to be in the dianionic state. The solution in which the chemiluminescent detection reaction takes place, *i.e.*, the porphyrin detection solution, preferably contains Tween-20 or linoleic acid, as described in Motsenbocker *et al., supra.* The Tween-20 and linoleic acid serve to increase the detectable chemiluminescent signal. In a preferred embodiment of the invention, potassium permanganate is heated and the resultant product is bubbled through a solution containing luminol, or a similar porphyrin detection reagent, so as to increase the sensitivity of the detection reaction. In an alternative preferred embodiment, potassium permanganate is directly added to the luminol solution.

The subject invention also provides novel methods of detecting a polynucleotide of interest based upon hybridization with a polynucleotide probe, wherein the polynucleotide probe is a porphyrin labeled polynucleotide of the invention as previously described. The polynucleotide of interest may be detected by the presence of the porphyrin label in the polynucleotide hybridization probe specific for the polynucleotide of interest. Preferably, the porphyrin labeled polynucleotide hybridization probe is detected on the basis of a detectable reaction product produced by an oxidation reaction catalyzed by the porphyrin moieties on the probe polynucleotide.

Porphyrin labeled polynucleotides may be used to hybridize to complementary target polynucleotides in essentially the same manner as polynucleotide hybridization probes in conventional nucleic acid hybridization techniques. Nucleic acid hybridization techniques are well known to the person of ordinary skill in the art and detailed descriptions of these techniques can be found, for example, in Sambrook, *et al.*, Molecular Cloning, A Laboratory Manual, 2nd Edition, Cold Spring Harbor Press, Cold Spring Harbor (1989). The porphyrin labeled polynucleotides of the invention may be substituted for conventional polynucleotides (radioactively labeled or otherwise) in standard hybridization methods so as to provide the methods for detecting polynucleotides of interest and methods for producing porphyrin labeled polynucleotides.

Typically, the polynucleotide detection methods of the invention comprise the steps of mixing a porphyrin labeled polynucleotide of the invention with a composition suspected of containing a complementary polynucleotide of interest under nucleic acid hybridization conditions that permit the formation of duplexes between the porphyrin labeled polynucleotide and the polynucleotide of interest, *i.e.*, the target. The duplexes are formed by hybridization at regions of homology between the porphyrin labeled polynucleotide probe and the polynucleotide of interest. The porphyrin labeled polynucleotide and, hence, the target polynucleotide of interest hybridized to the porphyrin labeled polynucleotide probe, may be detected by adding a porphyrin detection reagent. The porphyrin detection reagent then contacts the porphyrin moieties on the labeled polynucleotide, resulting in the oxidation of the reagent, which is catalyzed by the porphyrin on the labeled polynucleotide. The oxidation of the porphyrin detection reagent forms one or more detectable reaction products. Detectable reaction products include colored compounds (in colorimetric reactions) and light (in chemiluminescent reactions). The polynucleotide detection methods of the invention may further comprise the step of detecting the reaction product. The composition to be probed for the polynucleotide target of interest may take a variety of forms; however, the composition for analysis is preferably a composition consisting essentially of polynucleotides or proteins that bind to polynucleotides. The subject methods of detecting target polynucleotides of interest may be adapted to a variety of uses such as diagnosis, the detection of microbial contaminants, forensic uses, and the like.

The porphyrin labeled polynucleotides of the invention may also be used as primers in primer mediated cyclic amplification techniques employing a hybridization step, such as the polymerase chain reaction, ligase chain reaction, and strand displacement amplification, Guatelli et al., Proc. Nat. Acad. Sci. 87:1874-1878 (1990); Walker et al., Nucl. Acids Res. 20:1691-1696 (1992), *i.e.*, the labeled primer anneals (hybridizes) to the target sequence. For example, polynucleotides of interest may be detected by performing PCR (or similar amplification techniques) in which porphyrin labeled or unlabeled primer(s) are used to prime synthesis of a porphyrin labeled polynucleotide by incorporation of porphyrin labeled nucleotides. Alternatively, porphyrin labeled primer(s) are used to prime synthesis of a polynucleotide by incorporation of unlabeled nucleotides. The porphyrin labeled polynucleotide synthesized during amplification may be used to indicate the presence or absence of the polynucleotide of interest.

The porphyrin labeled polynucleotides of the invention may also be used to detect DNA sequence information produced using Sanger-type sequencing technology. For example, porphyrin-labeled polynucleotides may be used as sequencing primers, or porphyrin labeled dideoxynucleotides may be used as chain elongation terminators. Bands of interest formed in the sequence gel (or other suitable separation means) may be detected by incubating the sequencing gel with a porphyrin detection solution, followed by exposure to film (or other suitable detection means) so as to detect the bands. Alternatively, separated labeled polynucleotides produced by the sequencing reactions may be transferred to a solid support membrane in order to expedite detection of the sequence information.

The present invention also provides for methods of detecting porphyrin labeled polynucleotides that do not require a hybridization step. For example, in some embodiments of the invention, the detection method may be applied to the detection of a porphyrin labeled polynucleotide that is not hybridized to a target polynucleotide of interest, e.g. when a porphyrin labeled polynucleotide molecular weight size marker is used there is no need to perform a hybridization step.

When the porphyrin detection reagent is oxidized in a chemiluminescent reaction, the light produced by the reaction catalyzed by the porphyrin moieties on the labeled polynucleotide may be detected by a variety of means well known to the person of ordinary skill in the art. Suitable light detection means include photographic films, charge coupled devices, chemiluminometers, and the like. The amount of light produced by the chemiluminescent reaction may be measured quantitatively or qualitatively. Similarly, the amount of the product of a colorimetric reaction may be measured quantitatively or qualitatively by various well known instruments and techniques.

Another aspect of the invention is to provide kits for synthesizing porphyrin labeled polynucleotides using the method of the invention. The kits of the invention provide one or more of the compounds of the invention and one or more other reagents for use in performing the subject labeling methods. Kits may contain the subject compounds and reagents in pre-measured amounts so as to ensure both precision and accuracy when performing the subject methods. Kits may also contain instructions for performing the methods of the invention. Kits of the invention may typically contain one or more of the following items: porphyrin labeled nucleotides of the invention, unlabeled nucleotides (including mixtures of dATP, dCTP, dGTP and TTP), enzymes for catalyzing the polymerization of nucleotides (including the Klenow fragment of DNA polymerase I, Taq DNA polymerase, Pfu DNA polymerase, terminal transferase, and the like) buffers, porphyrin-labeled size markers, porphyrin detection reagents (including luminol) and the like.

Similarly, the invention provides kits for detecting polynucleotides of interest using porphyrin labeled polynucleotides as hybridization probes. The kits of the invention provide one or more of the porphyrin labeled polynucleotides and one or more other reagents for use in performing the subject polynucleotide detection methods. Kits may contain the compounds of the invention and reagents in pre-measured amounts so as to ensure both precision and accuracy when performing the subject methods. Kits may also contain instructions for performing the methods of the invention. Kits of the invention typically contain one or more of the following items: porphyrin labeled polynucleotides of the invention, porphyrin detection reagents (including luminol), polynucleotide hybridization reagents, polynucleotide immobilization membranes, and the like.

The invention having been described above, the following examples are offered in order to facilitate the understanding and practice of the invention. The following examples are not presented in order to limit the invention in any way.

### EXAMPLES

### Example 1

### Synthesis of amino-4-dUTP

400 mg (1.1 mmole) of 5-(trifluoroacetyl-4amino-propynyl)-2'-deoxyuridine (Hobbs, F.W., J. Org. Chem., 1989, 54, 3420-3422 or U.S. Patent 5,047,519) was dissolved in 30 ml of dry trimethylphosphate (TMP), and treated with 314 µL (3.3 mmole) of POCl₃ and 474 mg (2.2 mmole) of 1,8-*bis*-dimethylamino-naphthalene. The reaction was stirred at -5 °C under argon for 5 hours whereupon a cold (-10°C) solution of tributylammonium pyrophosphate (1.0 M, 4.2 ml) in TMP and tri-N-butylamine (2.5 ml) was added followed 15 minutes later by an addition of 50 ml of 0.5 M triethylammonium bicarbonate (TEAB). The resulting crude material was loaded onto a DEAE Sepharose ion exchange column (4x40cm) and eluted with a linear gradient of 0.0 - 0.4M TEAB. Appropriate fractions were pooled, concentrated *in vacuo,* and treated with 200 ml of 7M ammonium hydroxide at room temperature for 3 hours. The reaction contents were then evaporated to dryness and resuspended in deionized water. The nucleotide, m.s. (FAB-) 520 (M-H⁺), was of sufficient purity for subsequent coupling reactions.

### Example 2

### Synthesis of Fmoc-6-aminocaproic acid, N-hydroxysuccinimidyl ester

To a stirred suspension of 6-aminocaproic acid (13 mg, 100mmole) in a mixture of 70 ml of 100mM sodium bicarbonate and 200 ml acetonitrile was added N-(9-fluorenylmethoxycarbonyloxy)succinate (Fmoc-NHS, 5 g, 14 mmol, Aldrich) and the resulting suspension was stirred for two hours at room temperature. The reaction mixture was then evaporated to dryness, dissolved in dichloromethane and purified by silica gel column chromatography using dichloromethane:methanol (10:1) to yield 5.34 g of Fmoc-6-aminocaproic acid.

To 350 mg (1 mmol) of Fmoc-6-aminocaproic acid in 20 ml of DMF was added N-hydroxysuccinimide (230 mg, 2 mmol) and dicyclohexylcarbodiimide (412 mg, 2 mmol) in 10 ml of DMF. The solution was stirred overnight at room temperature and formed a gradual suspension. The mixture was concentrated *in vacuo* to a volume of 5 ml which was sufficiently pure for use in the following coupling step.

### Example 3

### Synthesis of Amino-11-dUTP

Amino-4-dUTP (100mg, 100µmol) was dissolved in 0.1 M aqueous sodium borate (25 ml, pH 9) and combined with 5 ml of the Fmoc-aminocaproic acid N-hydroxysuccinimidyl ester, described in Example 2. The mixture was stirred for 6 hours at room temperature and centrifuged at 2000 X g for 5 minutes. The solid pellet containing Fmoc-protected nucleotide was treated with a mixture of morpholine and DMF (1:1, 100ml) at room temperature for 6 hours, evaporated to near dryness and isolated by preparative reverse phase HPLC, using a C-18 column (7µm, 10 X 250 mm, Sherisorb) and 0.1M triethyammonium bicarbonate (TEAB) and 60% acetonitrile as solvents (called A and B, respectively). At a flow rate of 3 ml per minute, the column was eluted for 10 min at 2.5% B, followed by a gradient (increased to 65% B over 50 minutes). The desired product eluted from the column at a retention time of 20.4 minutes.

### Example 4

### Synthesis of Mn tetrakis (carboxyphenyl)porphyrin-12-dUTP

Sulfo-N-hydroxysuccinimide (25 mg, 115 µmol) and ethyl-3-(3-dimethylaminopropyl) carbodiimide (EDAC, 25 mg, 130 µmol) were added to 8 ml of 25 mM sodium phosphate buffer, pH 6. To this solution was added Mn *tetrakis*(carboxyphenyl) porphin chloride (6 mg, 6.8 µmol, Porphyrin Products, Logan, Utah) in 4.5 ml DMF. The reaction was allowed to stir at room temperature for two hours whereupon one ml of an aqueous solution containing 5 mg of amino-11-dUTP (5 µmol) was added and the solution was allowed to stir at room temperature in the dark. The porphyrin nucleotide was isolated by preparative reverse-phase HPLC using 0.1M triethyammonium bicarbonate (A) and 60% acetonitrile (B) as solvents. The column (10 x 250 mm) was eluted for 10 min at 2.5% B, followed by a gradient (increased to 65% acetonitrile over 50 minutes). The desired product had a retention time of 19.9 minutes. Fractions containing the product were combined, evaporated to dryness *in vacuo,* and coevaporated with aqueous ethanol (3 x 100 ml) to yield 1.3 mg of porphyrin labeled nucleotide (A₄₇₀ = 96,000).

### Example 5

### Synthesis of Mn tris(sulfonatophenyl) carboxyphenylporphyrin-12-dUTP (Method A)

Sulfo-N-hydroxysuccinimide (25 mg, 115 µmol) and EDAC (25 mg, 130 µmol) were added to 8 ml of 25 mM sodium phosphate buffer, pH 6. To this solution was added Mn *tris*(sulfonato phenyl) carboxyphenylporphin chloride (6 mg, 6.8 µmol, Porphyrin Products) in 4.5 ml DMF. The reaction was allowed to stir at room temperature for two hours whereupon one ml of an aqueous solution containing 5 mg of amino-11-dUTP (5 µmol,) was added and the solution was allowed to stir for 24 hours at room temperature in the dark. Analytical HPLC analysis of the reaction mixture showed a new product with an elution time of 30.7 minutes. The product, Mn *tris*(sulfonato phenyl) carboxyphenylporphyrin-12-dUTP, was isolated by reverse-phase HPLC by the method described in the Example 3.

### Example 6

### Synthesis of Mn tris(sulfonatophenyl)carboxyphenyl porphyrin-N-hydroxysuccinimidyl ester

Mn-monocarboxyphenyl-*tris*(sulfonatophenyl) porphin chloride (50 mg, 49 µmol, Porphyrin Products) was dissolved in 10 ml of dry pyridine and cooled to 0°C in an ice bath. A mixture of N-hydroxysuccinimide (11.4 mg, 0.1 mmol) and dicyclohexylcarbodiimide (20.6 mg, 0.1 mmol) in 1 ml of pyridine was added and the solution stirred at 0°C for 4 hours. The course of the reaction was monitored by analytical reverse phase HPLC (4.6 X 250 mm C-18 column at a flow rate of 1 ml/min) under the same gradient conditions described in Example 3. After 10 days the reaction contents were removed under vacuum and stored in the dark with exclusion of moisture. The product was sufficiently pure for use in subsequent coupling reactions.

### Example 7

### Synthesis of Mn tris(sulfonatophenyl)carboxyphenyl porphyrin-6-aminocaproic acid, N-hydroxysuccinimidyl ester

The residue from example 6 was dissolved in 2 ml of DMF, and added to a stirring solution of 27 mg (200 µmol) of 6-aminocaproic acid in 0.1M sodium borate, pH9. The solution was stirred for 2.5 hours at room temperature and isolated by preparative HPLC as described in example 3. The product eluted at a retention time of 29 minutes. m.s. (electrospray): 1063 (M-H⁺).

### Example 8

### Synthesis of Mn tris(sulfonatophenyl)carboxyphenyl porphyrin-12-dUTP (Method B)

The product from example 7 (50 mg) was dissolved in 10 ml of dry pyridine and combined with N-hydroxysuccinimide (NHS, 11.4 mg, 100 µmol) and the resulting solution was cooled to 0°C in an ice bath. Dicyclohexylcarbodiimide (DCC, 20.6 mg, 100 µmol) in 1 ml of dry pyridine was added and the reaction contents were stirred for 5 hours at 0°C whereupon additional NHS (11.4 mg) and DCC (21 mg) were added. After stirring for 40 hours, the contents were evaporated to dryness, dissolved in 2 ml of DMF and combined with 40 mg (40 µmol) of amino-4-dUTP in 6 ml of 100 mM sodium borate buffer, pH 9, and the contents stirred overnight at room temperature. The desired product was isolated by preparative HPLC to yield 15.4 mg of nucleotide which was identical to the product obtained by method A (Example 5) as determined by HPLC analysis and functional assays, *e.g.* polymerase catalyzed primer extension assay (Example 17).

### Example 9

### Synthesis of Mn tetrakis(carboxyphenyl)porphyrin-4-dUTP

Sulfo-N-hydroxysuccinimide (8 mg, 38 mmol) was dissolved in 4 ml of 25 mM aqueous sodium phosphate, pH 6.0 and combined with 6 mg of Mn *tetrakis*(carboxyphenyl)porphin chloride (6,8 µmol, Porphyrin Products) in 4 ml of DMF and 25 mg of ethyl-3-(3-dimethylaminopropyl)carbodiimide (EDAC, 130 µmol) in 4 ml of the same sodium phosphate buffer. The reaction was stirred in the dark at room temperature for 1.5 hr and then added to a solution of amino-4-dUTP (5 mg, 5 µmol,) in one ml of sodium phosphate buffer. The solution was stirred for 16 hours at room temperature and isolated by preparative HPLC as described for the other porphyrin nucleotides.

### Example 10

### Synthesis of Mn tris(sulfonatophenyl-meso-15-amino 4, 7, 10 trioxatridecan-1-carboxamidophenylporphyrin

Mn *tris*(sulfonato phenyl)carboxyphenylporphyrin chloride (30 mg, 30 µmol, Porphyrin Products) was dissolved in pyridine (15 ml, dried over 4Å molecular sieves). To this solution was added N-hydroxysuccinimide (24 mg., 210 µmole) and dicyclohexylcarbodiimide (54 mg, 260 µmole) in 4 ml of dry pyridine and the solution was stirred at 0°C in the dark for one hour and kept in the refrigerator for 4 days. The solvent was removed under vacuum and the remaining product was dissolved in 5 ml of DMF and added dropwise to a stirring solution of 4, 7, 10-trioxatridecane-1, 13-diamine (600 mg, 2.7 mmole, BASF) in 25 ml of 100 mM aqueous sodium borate (pH9). The reaction contents were stirred for one hour at 0°C and stored overnight at 5°C in the dark. The product was isolated by preparative reverse-phase HPLC using 0.1M triethyammonium bicarbonate (A) and 60% acetonitrile (B) as solvents. The column (10 X 250 mm, 7µm) was eluted for 10 min at 2.5% B, followed by a gradient (up to 65% B over 50 minutes). Fractions containing the product were combined, evaporated to dryness *in vacuo,* and coevaporated with aqueous ethanol (3 X 100 ml) to yield 45 mg of porphyrin amine. MS (electrospray) : 1154 (M + H⁺),
1176 (M + Na⁺), 1198 (M- H⁺ 2Na⁺).

### Example 11

### Synthesis of Mn tris(sulfonato phenyl)-15-succinoylamino 4, 7, 10 trioxatridecan-meso-1-carboxamidophenylporphyrin

The product from Example 10 (20 mg) in 10 ml of dry DMF was combined with succinic anhydride (50 mg, 0.5 mmole). The reaction contents were stirred at room temperature for 18 hours and the product was isolated by preparative HPLC as described in Example 10, above. MS(electrospray): 1252 (M-H+).

### Example 12

### Synthesis of Mn tris(sulfonato phenyl)-N-hydroxysuccinimidyl-15-succinoylamino 4, 7, 10 trioxatridecan-meso-1-carboxamidophenylporphyrin

The product from Example 11 (20 mg) was dissolved in 8 ml of dry pyridine and cooled to 0°C in an ice bath. To the stirred solution was added N-hydroxysuccinimide (24 mg, 210 mol) and dicyclohexylcarbodiimide (50 mg, 250 µmol) in 2 ml of dry pyridine. The solution was stirred at 0°C for six hours and stored at 4°C for three days whereupon the solution was evaporated to dryness. The product was suitable for use in subsequent coupling reactions.

### Example 13

### Synthesis of tris(sulfonato phenyl)-carboxyphenyl porphyrin-24-dUTP

The product from Example 11 was dissolved in 2 ml of dry DMF and combined with 20 mg (21 µmole) of amino-4 dUTP in 6 ml of aqueous sodium borate (100mM, pH 9) and stirred at room temperature for 24 hours. The nucleotide product was purified using an analytical HPLC column (5µ,C-18, 4.6 X 250 mm) at a flow rate of 1 ml per minute using the same buffers and gradient profile described previously. The appropriate HPLC fractions were pooled, evaporated to dryness and coevaporated several times with ethanol to decompose the excess TEAB salts.

### Example 14

### Synthesis of meso-tris(sulfonato phenyl)-carboxyphenylporphyrin-12-ddUTP

The product from example 7 (10 mg) was dissolved in 8 ml of pyridine and cooled to 0°C degrees. 6 mg of N-hydroxysuccinimide (NHS) and 12 mg of DCC were combined in 2 ml of pyridine and subsequently added to the porphyrincaproic acid solution. The mixture was stored in the refrigerator for 2 days whereupon DCC and NHS were again added, as before. After an additional day of reaction, 12 mg of NHS and 24 mg of DCC were added and allowed to react for an additional 24 hours. The reaction contents were then evaporated to dryness, dissolved in 1.2 ml of dry DMF and added with stirring to a solution of 17 mg of 5-aminopropynyl-2', 3'-dideoxyUTP (Hobbs, F.W. J. Org. Chem. 54, 3420-3422 (1989)) in 4 ml of 0.1 M aqueous sodium borate (0.1M, pH 9). The coupling reaction was allowed to proceed for 18 hours at room temperature after which the dideoxy nucleotide product was isolated by reversed phase HPLC following the isolation procedure described in Example 13.

### Example 15

### Synthesis of meso-tris(sulfonatophenyl)-carboxyphenylporphyrin-24-ddUTP

The meso-*tris*(sulfonato phenyl)-carboxyphenyl porphyrin-24-dd-UTP was synthesized following the procedure for the synthesis of the corresponding deoxynucleotide (Example 13) substituting aminopropynyl-ddUTP (amino-4-ddUTP, Hobbs, F.W. J. Org. Chem. 54, 3420-3422 (1989)), for amino-4dUTP. Isolation and purification procedures were similar to those described in Example 13.

### Example 16

### Measurement of Incorporation Of Porphyrin-12-dUTP in Randomly Primed Synthesis

Method 1: Linearized pBluescript DNA was labeled using random nonamers, unmodified nucleotides, ³H dCTP, and various ratios of TTP/porphyrin-12-dUTP. Template DNA (pBluescript DNA linearized with XhoI, lOng) and random nonamers (6.8µg) were combined, denatured by boiling for 5 minutes and allowed to cool to room temperature. To each reaction was added Tris buffer (pH 7.5), MgCl₂ and dithiothreitol (to final concentrations of 35 mM, 5 mM and 1 mM, respectively). Nucleotide mixtures were added (each at a final concentration of 20 µM) containing dATP, dGTP, dCTP (including 1 µL of 0.1 mCi/ml ³H dCTP) and various mixtures of porphyrin-dUTP and TTP (in ratios of 0:100, 25:75, 50:50, 75:25, and 100:0, respectively). Exonuclease free Klenow polymerase (5U) was added and the mixtures were incubated at 37°C for 10 minutes. Aliquots (4 µL) were spotted onto DE 81 discs, dried under a heat lamp for 15 minutes, washed in 2X SSC (6 X 3 minutes), washed in cold ethanol (1 minute), and the relative radioactive levels were determined in comparison with unwashed controls by scintillation counting. Reactions were performed in triplicate.

Incorporation levels of ³H dCTP were highest in those reactions where porphyrin-dUTP:TTP ratios were 25:75 and 50:50 and were equivalent to the (maximal) incorporation levels observed in those samples where no porphyrin dUTP was included (porphyrin-dUTP:TTP = 0:100). Incorporation levels of the samples containing 75:25 (porphyrin-dUTP:TTP) were observed to be about 70% of maximal.

### Example 17

### Primer Extension Assays Using Porphyrin 12-dUTP

The -20 primer (M13) was end labeled with ³²P ATP and T4 polynucleotide kinase, annealed to a complementary 80-mer template, extended in the presence of nucleotides (including porphyrin-12-dUTP) and various polymerases and the results were analyzed by 10% PAGE. The enzymes tested included, among others: Taq™, Pfu™ and exonuclease free Pfu, Vent™, Sequenase™, and exonuclease free Klenow DNA polymerases. Primers were kinased using ³²P ATP (10 mCI/ml, DuPont/NEN) and polynucleotide kinase (as described in Sambrook *et al.,* Molecular Cloning Manual, 2nd edition, 10.59 - 10.66). Aliquots (10 ng) of 5'-labeled primers were combined with 100ng of complementary 80-mer template, 10 µM solutions (each) of dATP, dCTP and dGTP, 20 µM porphyrin-12-dUTP, and the reaction buffer appropriate for each polymerase. The reactions were heated briefly to 90°C, slowly cooled to reaction temperature, and 5 units of polymerase were added. The reactions containing Sequenase™ and exonuclease free Klenow were allowed to react at 37°C for 15 minutes. All other incubations were at 72°C. The reactions were terminated by addition of 3 µL of a solution containing 95% formamide, 0.1% bromophenyl blue, 0.2 M EDTA and aliquots were loaded onto a 10% acrylamide gel and electrophoresed for 3 hours at 55 watts/2250 mV. Extensions were observed in reactions using each of the enzymes. Some full length extension was observed for all enzymes except Vent™ polymerase. Optimal extensions were observed with Taq DNA polymerase and exonuclease free Pfu™ DNA polymerase.

### Example 18

### Porphyrin incorporation into DNA via PCR

Each PCR reaction (50 µL, final volume) contained 5 µL Taq DNA polymerase reaction buffer, 20 ng of Bluescript KS+, 125 ng each of M13 reverse and -20 primers, 7.5 µL of 1mM each of dGTP, dATP and dCTP, 4.5 µL of 1mM TTP, and 5µL of 600 µM porphyrin-12-dUTP. Taq DNA polymerase (5U) was added to each reaction. Reactions were heated as follows: 95°C for 45 seconds, 50°C for 90 seconds and 72°C for 90 seconds, repeated for 30 cycles. Negative control reactions were run which contained 5µL of 600 µM porphyrin-dUTP but no Taq polymerase, in order to check for background signal due to nonspecific intercalation of the porphyrin-dUTP into the control DNA. The full length PCR products were purified by loading the reaction mixture (50 µL) atop a column containing 2 ml of Bio Gel P60, eluting with a low salt buffer (15mM NaCl and 15 mM Tris buffer, pH 7.5), and collecting the effluent into 125µL fractions. Aliquots from each column were loaded onto a 1% agarose gel, electrophoretically separated at 90mA for one hour and visualized after staining with ethidium bromide. The gel was then briefly treated with 0.25M HCl (3 minutes), 1.5M NaCl-0.5M NaOH (3 minutes) and then 0.1M Tris pH 7.5 - 1.5M NaCl (3 minutes), and transferred onto Pall Biodyne (N+) membrane via capillary techniques. The nucleic acid components were immobilized onto the membrane using UV light, using a Stratalinker™ UV crosslinker (Stratagene, La Jolla, CA), on the autocrosslink setting. The membrane was then washed in deionized water for 1 minute, dried, sprayed with a detection buffer consisting of 5 mM luminol, 1% linoleic acid and 0.1M (aqueous) NaOH, pH13 covered with clear plastic wrap and immediately exposed to film.

Film exposure was performed for 5 minutes. No chemiluminescent signal was detected from bands of DNA made in the absence of the porphyrin label or in the absence of Taq DNA polymerase, but chemiluminescent signal was detected in the PCR products made in the presence of porphyrin-dUTP.

### Example 19

### Detection of Porphyrin-Labeled DNA

The porphyrin detection solution was made as follows: Luminol (270 mg, 1.5 mmole, Aldrich) was added to 300 ml of 0.1 M aq NaOH. Additional base (6N NaOH) was added until complete dissolution of luminol. Linoleic acid (3.3 g, 11.8 mmole, Aldrich) was then added and the mixture was shaken to dissolve the linoleic acid. Additional 6 N NaOH was added to bring the solution to pH 13.

Enhanced porphyrin detection solutions were made by one of the following methods: (a) The luminol buffer from above (unenhanced) was cooled to 0°C and product obtained by heating solid potassium permanganate was bubbled into the solution for 5 minutes with stirring. Alternatively (b), to 50 ml of the unenhanced luminol buffer (above) was added 5 mg, (32 µmole) of potassium permanganate. The enhanced buffers were used in the detection of serial dilutions of porphyrin-labeled DNA probe (dot blots) and showed a sensitivity of at least 10 fold higher than the unenhanced solutions.

The detection solutions were used to detect PCR amplification products described in Example 18.

### Example 20

### Porphyrin Labelled DNA Probes in Southern Hybridizations

**1. Probe Labeling**
   200ng aliquots of pBluescript DNA linearized with XhoI were labeled with porphyrin-12-dUTP by random priming as follows: 200ng template DNA were denatured by boiling for 5 minutes with 6.75µg random nonamer primers in a total volume of 39µL. The reaction tube was placed on ice and 10µL of 5X nucleotide buffer (100 µM each dATP, dCTP, dGTP, and either 100µM porphyrin-12-dUTP or 90 µM porphyrin-12-dUTP and 10 µM TTP in 175mM Tris pH 7.5, 25mM MgCl₂) and 10u of exonuclease free Klenow polymerase were added. The mixture was incubated at 37°C for 1 hour and the reaction was terminated with 15mM EDTA. Probe fragments were purified from unincorporated nucleotides using a BioGel P60 drip column equilibrated with 25 mM Tris-HCl, pH 7.5, 25mM NaCl. The reaction mixture (50µL) was loaded onto a 2 ml column and 4 X 340 µL aliquots of equilibration buffer were added. The eluted fractions were collected separately and assayed for porphyrin activity by spotting 1µL aliquots onto Pall Biodyne Plus nylon membrane, crosslinking with the Stratagene UV crosslinker and submerging briefly in 5 mM luminol, 1.1% linoleic acid, pH 13.0 before exposing to X-ray film for 15 minutes. The spotted aliquot from fraction 2 produced the greatest signal and this fraction was used as the source of porphyrin probe polynucleotide in the subsequent hybridization experiments.
**2. Hybridization of Probe to Target DNA**
   Dilutions of target pBluescript DNA (lOng, 1ng, 500pg, 100pg, 50pg and 10pg) were denatured by boiling, spotted onto Biodyne Plus membrane, and immobilized by UV light (Stratalinker™, autocrosslink setting). Aliquots of salmon sperm DNA (1µg and 100 ng) were similarly immobilized on the membrane which was then prehybridized in 1.5X SSPE, 3% SDS, 10% PEG for 30 minutes at 68°C. Porphyrin labeled probe was denatured by boiling for 5 minutes, added to the prehybridization buffer at a final concentration of 300ng starting template per ml of hybridization buffer and the resulting mixture was allowed to hybridize overnight at 68°C. The membranes were washed in 0.1X SSC/0.1% SDS at room temperature for 15 minutes and twice (15 minutes each) at 60°C with the same buffer. The membranes were rinsed in TBS (50 mM Tris-HCl pH 7.5, 150 mM NaCl) at room temperature twice for 5 minutes each time. The luminol solution (described in Example 19) was spread carefully over the surface of each membrane. Excess luminol solution was blotted from the membrane which was then covered with plastic wrap and exposed to X-ray film.

Signal was obtained from the pBluescript DNA spots in all cases but not from the control salmon sperm DNA spots. After a 30 minute exposure to film, probe made with 100% porphyrin nucleotide substitution (no TTP in the reaction buffer) allowed detection of at least 50 pg of target pBluescript DNA while probe made with 90% porphyrin (with 10% TTP in the reaction buffer) allowed detection of at least 10 pg of target DNA.

### Example 21

### Porphyrin Labelled DNA Markers

DNA size markers (Lambda Hind III digest, 2 µg) were labeled using Taq polymerase (5U) in 10 µL of 5X nucleotide buffer (100 µM each dATP, dCTP, dGTP, and 90 µM porphyrin-12-dUTP and 10 µM TTP in 25 mM Tris HCl pH7.5, 100mM KCl buffer). The mixture was incubated at 60°C for 10 minutes; the tube condensate was spun down by a brief centrifugation and the reaction was again heated at 60°C for 10 minutes. The reaction mixture was ethanol-precipitated, and the precipitate was suspended in 50 µL of water and purified using a P60 drip column as described in Example 20. The eluted fractions which gave a positive chemiluminescent signal (assayed as described in Example 20, above) were pooled, electrophoresed on a 0.8% agarose gel and stained with ethidium bromide. The gel was then transferred onto a Pall Biodyne Plus nylon membrane. The membrane was irradiated with UV light (using a Stratalinker on the autocrosslink setting), washed in deionized water for 1 minute, dried, sprayed with the luminol detection buffer (described above) and immediately exposed to film. After a 5 minute exposure, a ladder of porphyrin labeled DNA size markers was observed that was identical to that observed after staining with ethidium bromide.

### Example 22

### Chain Termination

The porphyrin dideoxynucleotide (porphyrin-12-ddUTP) was tested for utility as a chain terminator in the following manner:
800 ng(4µL) of M-13 DNA was chain terminated by following the Sequenase™ protocol using the universal -20 primer (10 ng) and the Sequenase™ buffer and labeling mixes (United States Biochemicals). To demonstrate chain termination with porphyrin-12-ddUTP, termination mixes were made up with dGTP, dCTP and dATP (80 µM each) in 50 mM NaCl and ddTTP (8µM, as a control) or porphyrin-12-ddUTP at a concentration of either 20 µM, 80 µM, or 160 µM. Labeling and termination steps for all samples were carried out according to the manufacturer's protocol whereby α³³P-dATP is incorporated during the labeling step. Samples were electrophoresed on a 6% denaturing polyacrylamide gel. The sample containing 20µM porphyrin-12-ddUTP showed a banding pattern similar to the control which contained no porphyrin-12-ddUTP.

## Claims

1. A compound having the formula: porphyrin-linker-base-sugar-(phos)ₙ-OH, wherein n is 0 to 5, and phos is selected from the group consisting of phosphate, phosphorothioates, phosphonates, and structures represented by the formula: 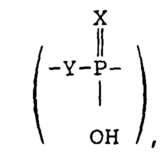 wherein X is O or S,
and Y is selected from the group consisting of O, S, CH₂-, CF₂- and NH-, wherein the "-" represents a covalent linkage between the indicated moieties of said compound.

2. A compound according to Claim 1, wherein the sugar is selected from the group consisting of ribose, deoxyribose, 2',3' dideoxyribose, carbocyclic ribose analogs, acyclic analogs of ribose, and heterocyclic analogs of ribose.

3. A compound according to claim 2, wherein the compound is a nucleotide.

4. A compound according to Claim 3, wherein the base moiety is selected from the group consisting of adenine, thymine, uracil, guanine, cytosine, or functionally equivalent heterocyclic analogs thereof.

5. A compound according to Claim 4, wherein said heterocyclic analog is selected from the group consisting of azapurines, azapyrimidines, deazapurines, deazapyrimidines, purine analogs containing oxygen heteroatoms, purine analogs containing sulfur heteroatoms, pyrimidine analogs containing oxygen heteroatoms, and pyrimidine analogs containing sulfur heteroatoms.

6. A compound according to Claim 2, wherein the linker backbone has a length of between 1 and 50 atoms.

7. A compound according to claim 3 wherein the compound is selected from the group consisting of
Mn-tetrakis(carboxyphenyl)porphyrin-12-dUTP;
Mn-tris(sulfonatophenyl)-carboxyphenylporphyrin-12-dUTP;
Mn-tris(sulfonatophenyl)-carboxyphenylporphyrin-24-dUTP;
Mn-tris(sulfonatophenyl)-carboxyphenylporphyrin-12-ddUTP;
Mn-tris(sulfonatophenyl)-carboxyphenylporphyrin-24-ddUTP; and
Mn-tris(sulfonatophenyl)-carboxyphenylporphyrin-12-dCTP.

8. A porphyrin labeled polynucleotide having a structure comprising a moiety having the formula of a compound according to Claim 1.

9. A porphyrin labeled polynucleotide having a structure comprising a moiety having a structure according to Claim 2.

10. A porphyrin labeled polynucleotide having a structure comprising a moiety having a structure according to Claim 3.

11. A porphyrin labeled polynucleotide having a structure comprising a moiety having a structure according to Claim 4.

12. A method of detecting a polynucleotide of interest, said method comprising the steps of:
a) mixing a porphyrin labeled polynucleotide according to claim 8 with a porphyrin detection reagent, wherein the porphyrin detection reagent produces a detectable signal when mixed with the porphyrin labeled polynucleotide; and
b) detecting said detectable signal.

13. A method of detecting a polynucleotide of interest, said method comprising the steps of
a) mixing a porphyrin labeled polynucleotide according to claim 8 with a composition suspected of containing the polynucleotide of interest wherein the porphyrin labeled polynucleotide has at least one region of sequence homologous to the polynucleotide of interest, whereby duplexes are formed;
(b) contacting the porphyrin labeled polynucleotide with a solution comprising a porphyrin detection reagent under conditions whereby a reaction product is formed;
(c) detecting the reaction product.

14. A method according to Claim 12, wherein the porphyrin detection reagent is a substrate in a chemiluminescent reaction.

15. A method according to Claim 13, wherein the porphyrin detection reagent is a substrate in a chemiluminescent reaction.

16. A method according to Claim 12, wherein the porphyrin detection reagent is a substrate in a colorimetric reaction.

17. A method according to Claim 13, wherein the porphyrin detection reagent is a substrate in a colorimetric reaction.

18. A method according to Claim 12, further comprising the step of adding at least one reagent capable of enhancing the detection of the reaction product.

19. A method according to Claim 13, further comprising the step of adding at least one reagent capable of enhancing the detection of the reaction product.

20. A method of detecting a polynucleotide of interest comprising the steps of:
a) hybridizing a polynucleotide primer to the polynucleotide of interest,;
b) admixing a mixture of nucleotides comprising a porphyrin labeled nucleotide according to Claim 3;
c) synthesizing a porphyrin labeled polynucleotide, wherein synthesis is initiated from said primer; and
d) detecting the porphyrin labeled polynucleotide.

21. A method of detecting a polynucleotide of interest, said method comprising the steps of
a) hybridizing a primer polynucleotide according to claim 8 to the polynucleotide of interest;
b) admixing a mixture of nucleotides, wherein the mixture can support polynucleotide synthesis;
c) synthesizing a porphyrin labeled polynucleotide, wherein synthesis is initiated from said primer; and
d) detecting the porphyrin labeled polynucleotide.

22. A method according to claim 21 wherein the mixture comprises a nucleotide according to Claim 1.

23. A kit for producing porphyrin labeled polynucleotides, said kit comprising a compound according to Claim 3, and an enzyme having polynucleotide polymerase activity.

24. A kit for detecting a polynucleotide of interest, said kit comprising a polynucleotide according to Claim 8 and a porphyrin detection reagent.

25. A compound according to Claim 1, wherein the porphyrin is one which catalyzes a chemiluminescent reaction.

26. A porphyrin labeled polynucleotide according to Claim 8, said labeled polynucleotide comprising a plurality of porphyrins.

27. A porphyrin labeled polynucleotide according to Claim 8, wherein the porphyrin is one which catalyzes a chemiluminescent reaction.

## Patentansprüche

1. Verbindung mit der Formel: Porphyrin-Linker-Base- Zucker-(Phos)ₙ-OH, worin n 0 bis 5 ist, und Phos gewählt ist aus der Gruppe, bestehend aus Phosphat, Phosphorthioaten, Phosphonaten und Strukturen, wie dargestellt durch die Formel: 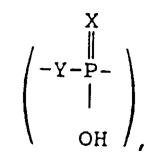 worin X ist O oder S, und Y gewählt ist aus der Gruppe, bestehend aus O, S, CH₂-, CF₂- und NH-, wobei der "-" für eine kovalente Bindung zwischen den angegebenen Komponenten der Verbindung steht.

2. Verbindung nach Anspruch 1, wobei der Zucker gewählt ist aus der Gruppe, bestehend aus Ribose, Desoxyribose, 2',3'-Didesoxyribose, carbocyclische Ribose-Analoga, acyclische Analoga von Ribose und heterocyclische Analoga von Ribose.

3. Verbindung nach Anspruch 2, wobei die Verbindung ein Nukleotid ist.

4. Verbindung nach Anspruch 3, wobei die Basenkomponente gewählt ist aus der Gruppe, bestehend aus Adenin, Thymin, Uracil, Guanin, Cytosin oder deren funktionell äquivalente heterocyclische Analoga.

5. Verbindung nach Anspruch 4, wobei das heterocyclische Analogon gewählt ist aus der Gruppe, bestehend aus Azapurinen, Azapyrimidinen, Deazapurinen, Deazapyrimidinen, Sauerstoffheteroatome enthaltende Purin-Analoga, Schwefelheteroatome enthaltende Purin-Analoga, Sauerstoffheteroatome enthaltende Pyrimidin-Analoga und Schwefelheteroatome enthaltende Pyrimidin-Analoga.

6. Verbindung nach Anspruch 2, wobei das Linker-Grundgerüst eine Länge von 1 bis 50 Atomen aufweist.

7. Verbindung nach Anspruch 3, wobei die Verbindung gewählt ist aus der Gruppe, bestehend aus
Mn-tetrakis(carboxyphenyl)porphyrin-12-dUTP;
Mn-tris(sulfonatophenyl)-carboxyphenylporphyrin-12-dUTP;
Mn-tris(sulfonatophenyl)-carboxyphenylporphyrin-24-dUTP;
Mn-tris(sulfonatophenyl)-carboxyphenylporphyrin-12-ddUTP;
Mn-tris(sulfonatophenyl)-carboxyphenylporphyrin-24-ddUTP; und
Mn-tris(sulfonatophenyl)-carboxyphenylporphyrin-12-dCTP;

8. Porphyrin-markiertes Polynukleotid mit einer Struktur, umfassend eine Komponente mit der Formel einer Verbindung nach Anspruch 1.

9. Porphyrin-markiertes Polynukleotid mit einer Struktur, umfassend eine Komponente mit einer Struktur nach Anspruch 2.

10. Porphyrin-markiertes Polynukleotid mit einer Struktur, umfassend eine Komponente mit einer Struktur nach Anspruch 3.

11. Porphyrin-markiertes Polynukleotid mit einer Struktur, umfassend eine Komponente mit einer Struktur nach Anspruch 4.

12. Verfahren zum Nachweisen eines Polynukleotids von Interesse, welches Verfahren die folgenden Schritte umfaßt:
a) Mischen eines Porphyrin-markierten Polynukleotids nach Anspruch 8 mit einem Porphyrin-Nachweisreagenz, wobei das Porphyrin-Nachweisreagenz ein nachweisbares Signal erzeugt, wenn mit dem Porphyrin-markierten Polynukleotid vermischt; und
b) Nachweisen des nachweisbaren Signals.

13. Verfahren zum Nachweisen eines Polynukleotids von Interesse, welches Verfahren die folgenden Schritte umfaßt:
a) Mischen eines Porphyrin-markierten Polynukleotids nach Anspruch 8 mit einer Zusammensetzung, in der ein Gehalt des Polynukleotids von Interesse vermutet wird, wobei das Porphyrin-markierte Polynukleotid mindestens eine zum Polynukleotid von Interesse homologe Sequenzregion aufweist, wobei Duplexe gebildet werden;
b) Zusammenbringen des Porphyrin-markierten Polynukleotids mit einer Lösung, umfassend ein Porphyrin-Nachweisreagenz unter Bedingungen, bei denen ein Reaktionsprodukt gebildet wird;
c) Nachweisen des Reaktionsprodukts.

14. Verfahren nach Anspruch 12, wobei das Porphyrin-Nachweisreagenz ein Substrat bei einer chemilumineszenten Reaktion ist.

15. Verfahren nach Anspruch 13, wobei das Porphyrin-Nachweisreagenz ein Substrat in einer Chemilumineszenzreaktion ist.

16. Verfahren nach Anspruch 12, wobei das Porphyrin-Nachweisreagenz ein Substrat in einer kolorimetrischen Reaktion ist.

17. Verfahren nach Anspruch 13, wobei das Porphyrin-Nachweisreagenz ein Substrat in einer kolorimetrischen Reaktion ist.

18. Verfahren nach Anspruch 12, außerdem umfassend den Schritt der Zugabe mindestens eines Reagenz, das zum Verbessern des Nachweises des Reaktionsprodukts in der Lage ist.

19. Verfahren nach Anspruch 13, außerdem umfassend den Schritt der Zugabe mindestens eines Reagenz, das zum Verbessern des Nachweises des Reaktionsprodukts in der Lage ist.

20. Verfahren zum Nachweisen eines Polynukleotids von Interesse, umfassend die folgenden Schritte:
a) Hybridisieren eines Polynukleotid-Primers an das Polynukleotid von Interesse;
b) Vermengen eines Gemischs von Nukleotiden, umfassend ein Porphyrin-markiertes Nukleotid nach Anspruch 3;
c) Synthetisieren eines Porphyrin-markierten Polynukleotids, wobei die Synthese vom Primer ausgelöst wird; und
d) Nachweisen des Porphyrin-markierten Polynukleotids.

21. Verfahren zum Nachweisen eines Polynukleotids von Interesse, wobei das Verfahren die folgenden Schritte umfaßt:
a) Hybridisieren eines Primer-Polynukleotids nach Anspruch 8 an das Polynukleotid von Interesse;
b) Vermengen eines Gemischs von Nukleotiden, wobei das Gemisch die Polynukleotid-Synthese fördern kann;
c) Synthetisieren eines Porphyrin-markierten Polynukleotids, wobei die Synthese vom Primer ausgelöst wird; und
d) Nachweisen des Porphyrin-markierten Polynukleotids.

22. Verfahren nach Anspruch 21, wobei das Gemisch ein Nukleotid nach Anspruch 1 umfaßt.

23. Kit zur Herstellung Porphyrin-markierter Polynukleotide, wobei der Kit eine Verbindung nach Anspruch 3 und ein Enzym mit Polynukleotid-Polymerase-Aktivität umfaßt.

24. Kit zum Nachweisen eines Polynukleotids von Interesse, wobei der Kit ein Polynukleotid nach Anspruch 8 und ein Porphyrin-Nachweisreagenz umfaßt.

25. Verbindung nach Anspruch 1, wobei das Porphyrin ein solches ist, das eine Chemilumineszenzreaktion katalysiert.

26. Porphyrin-markiertes Polynukleotid nach Anspruch 8, wobei das markierte Polynukleotid eine Vielzahl von Porphyrinen umfaßt.

27. Porphyrin-markiertes Polynukleotid nach Anspruch 8, wobei das Porphyrin ein solches ist, das eine Chemilumineszenzreaktion katalysiert.

## Revendications

1. Composé ayant la formule :
porphyrine-linker-base-sucre-(phos)ₙ-OH, dans laquelle n est 0 à 5, et phos est choisi dans le groupe constitué par un phosphate, les phosphorothioates, les phosphonates et les structures représentées par la formule : 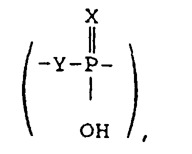 dans laquelle X est O ou S, et Y est choisi dans le groupe constitué par O, S, CH₂-, CF₂- et NH-, où les "-" représentent une liaison covalente entre les parties indiquées dudit composé.

2. Composé selon la revendication 1, dans lequel le sucre est choisi dans le groupe constitué par le ribose, le désoxyribose, le 2',3'-didésoxyribose, les analogues carbocycliques du ribose, les analogues acycliques du ribose et les analogues hétérocycliques du ribose.

3. Composé selon la revendication 2, le composé étant un nucléotide.

4. Composé selon la revendication 3, dans lequel la partie base est choisie dans le groupe constitué par l'adénine, la thymine, l'uracile, la guanine, la cytosine ou des analogues hétérocycliques fonctionnellement équivalents de ceux-ci.

5. Composé selon la revendication 4, dans lequel ledit analogue hétérocyclique est choisi dans le groupe constitué par les azapurines, les azapyrimidines, les désazapurines, les désazapyrimidines, les analogues de purine contenant des hétéroatomes oxygène, les analogues de purine contenant des hétéroatomes soufre, les analogues de pyrimidine contenant des hétéroatomes oxygène et les analogues de pyrimidine contenant des hétéroatomes soufre.

6. Composé selon la revendication 2, dans lequel le squelette du linker a une longueur entre 1 et 50 atomes.

7. Composé selon la revendication 3, le composé étant choisi dans le groupe constitué par :
Mn-tétrakis(carboxyphényl)porphyrine-12-dUTP ;
Mn-tris(sulfonatophényl)-carboxyphénylporphyrine-12-dUTP ;
Mn-tris(sulfonatophényl)-carboxyphénylporphyrine-24-dUTP ;
Mn-tris(sulfonatophényl)-carboxyphénylporphyrine-12-ddUTP ;
Mn-tris(sulfonatophényl)-carboxyphénylporphyrine-24-ddUTP ; et
Mn-tris(sulfonatophényl)-carboxyphénylporphyrine-12-dCTP.

8. Polynucléotide marqué par une porphyrine, ayant une structure comprenant une partie ayant la formule d'un composé selon la revendication 1.

9. Polynucléotide marqué par une porphyrine, ayant une structure comprenant une partie ayant une structure selon la revendication 2.

10. Polynucléotide marqué par une porphyrine, ayant une structure comprenant une partie ayant une structure selon la revendication 3.

11. Polynucléotide marqué par une porphyrine, ayant une structure comprenant une partie ayant une structure selon la revendication 4.

12. Procédé de détection d'un polynucléotide d'intérêt, ledit procédé comprenant les étapes consistant :
a) à mélanger un polynucléotide marqué par une porphyrine selon la revendication 8 avec un réactif de détection de porphyrine, le réactif de détection de porphyrine produisant un signal détectable lorsqu'il est mélangé avec le polynucléotide marqué par une porphyrine ; et
b) à détecter ledit signal détectable.

13. Procédé de détection d'un polynucléotide d'intérêt, ledit procédé comprenant les étapes consistant
a) à mélanger un polynucléotide marqué par une porphyrine selon la revendication 8 avec une composition suspectée de contenir le polynucléotide d'intérêt, le polynucléotide marqué par une porphyrine ayant au moins une région de séquence homologue de celle du polynucléotide d'intérêt, de sorte que des duplex se forment ;
(b) à mettre en contact le polynucléotide marqué par une porphyrine avec une solution comprenant un réactif de détection de porphyrine, dans des conditions telles qu'un produit de réaction se forme ;
(c) à détecter le produit de réaction.

14. Procédé selon la revendication 12, dans lequel le réactif de détection de porphyrine est un substrat dans une réaction chimioluminescente.

15. Procédé selon la revendication 13, dans lequel le réactif de détection de porphyrine est un substrat dans une réaction chimioluminescente.

16. Procédé selon la revendication 12, dans lequel le réactif de détection de porphyrine est un substrat dans une réaction colorimétrique.

17. Procédé selon la revendication 13, dans lequel le réactif de détection de porphyrine est un substrat dans une réaction colorimétrique.

18. Procédé selon la revendication 12, comprenant en outre l'étape consistant à ajouter au moins un réactif apte à améliorer la détection du produit de réaction.

19. Procédé selon la revendication 13, comprenant en outre l'étape consistant à ajouter au moins un réactif apte à améliorer la détection du produit de réaction.

20. Procédé de détection d'un polynucléotide d'intérêt, comprenant les étapes consistant :
a) à hybrider une amorce de polynucléotide au polynucléotide d'intérêt ;
b) à mélanger un mélange de nucléotides comprenant un nucléotide marqué par une porphyrine selon la revendication 3 ;
c) à synthétiser un polynucléotide marqué par une porphyrine, la synthèse étant déclenchée à partir de ladite amorce ; et
d) à détecter le polynucléotide marqué par une porphyrine.

21. Procédé de détection d'un polynucléotide d'intérêt, ledit procédé comprenant les étapes consistant
a) à hybrider un polynucléotide amorce selon la revendication 8 au polynucléotide d'intérêt ;
b) à mélanger un mélange de nucléotides, le mélange pouvant supporter la synthèse d'un polynucléotide ;
c) à synthétiser un polynucléotide marqué par une porphyrine, la synthèse étant déclenchée à partir de ladite amorce ; et
d) à detecter le polynucléotide marqué par une porphyrine.

22. Procédé selon la revendication 21, dans lequel le mélange comprend un nucléotide selon la revendication 1.

23. Nécessaire pour préparer des polynucléotides marqués par une porphyrine, ledit nécessaire comprenant un composé selon la revendication 3 et une enzyme ayant une activité de polynucléotide polymérase.

24. Nécessaire pour détecter un polynucléotide d'intérêt, ledit nécessaire comprenant un polynucléotide selon la revendication 8 et un réactif de détection de porphyrine.

25. Composé selon la revendication 1, dans lequel la porphyrine est une porphyrine qui catalyse une réaction chimioluminescente.

26. Polynucléotide marqué par une porphyrine selon la revendication 8, ledit polynucléotide marqué comprenant plusieurs porphyrines.

27. Polynucléotide marqué par une porphyrine selon la revendication 8, dans lequel la porphyrine est une porphyrine qui catayse une réaction chimioluminescente.
